# EUROPEAN PATENT APPLICATION

(11) **EP 4 674 810 A1**
(43) Date of publication of application: **07.01.2026**
(21) Application number: 24763077.5
(22) Date of filing: 26.02.2024
(51) Int. Cl.: C01B 39/22, B01J 20/18, B01J 20/30

(54) **MODIFIED TYPE X MOLECULAR SIEVE, ADSORBENT CONTAINING MODIFIED TYPE X MOLECULAR SIEVE, AND PREPARATION METHODS FOR AND USES OF TYPE X MOLECULAR SIEVE AND ADSORBENT**

(30) Priority: 27.02.2023 CN 202310170490
(71) Applicant: China Petroleum & Chemical Corporation, Beijing 100728 (CN); Sinopec Research Institute of Petroleum Processing Co., Ltd., Beijing 100083 (CN)
(72) Inventor: GAO, Ningning, Beijing 100083 (CN); WANG, Huiguo, Beijing 100083 (CN); ZHONG, Jin, Beijing 100083 (CN); TUO, Pengfei, Beijing 100083 (CN); GAO, Junkui, Beijing 100083 (CN); CHEN, Tiantian, Beijing 100083 (CN)
(74) Representative: karo IP
(86) International application number: PCT/CN2024/078502
(87) International publication number: WO 2024/179395

(57) **Abstract**

The present invention relates to a modified X-type molecular sieve, wherein the surface area of the modified X-type molecular sieve is 9.5-19 m²/g, and the sum of the mesopore and macropore volumes accounts for 6%-18% of the total pore volume; the present invention also relates to an adsorbent for aromatic hydrocarbon isomer containing the modified X-type molecular sieve, and their preparation method and use. The modified X-type molecular sieve and the adsorbent are used for adsorptive separation of p-xylene from mixed C8 aromatic hydrocarbons.

## Description

### Technical Field

The present invention relates to the technical field of adsorptive separation of organic matter, and more specifically to a modified X-type molecular sieve and an adsorbent containing the modified X-type molecular sieve as an active component, and their preparation methods and uses.

### Background Art

Aromatic monomers are an important type of petrochemical basic raw materials with high product added value. They can be widely used in the production of polyester, pesticides, medicines and other fields. In the industrial production process, the aromatic hydrocarbon isomers with multiple substituents are usually produced in the form of a mixture, and further separation is required to obtain monomers with high added value. Since these aromatic hydrocarbon isomers have very close boiling points, traditional distillation processes cannot separate and purify them. Therefore, selective adsorption methods are currently widely used in industry to separate them. The most widely used scenario for this technology is the separation of p-xylene from mixed C₈ aromatic hydrocarbons.

X-type molecular sieve adsorbents exchanged with barium ions or barium and potassium ions have the property of selectively adsorbing p-xylene. Through the simulated moving bed process, the mixed C8 aromatic hydrocarbons and the adsorbents are repeatedly adsorbed and desorbed to enrich p-xylene in the adsorbents, and then highly-pure p-xylene is obtained through desorption and distillation operations. Wherein, the preparation of high-performance adsorbents is the key to obtaining highly-pure p-xylene products.

The active component used as the adsorbent for the adsorptive separation of p-xylene in industry is mostly X-type molecular sieve. The X-type molecular sieve is mixed evenly with clay as a binder in a certain proportion, and the adsorbent pellets are obtained after ball-rolling, drying, calcination and cation exchange. Adsorption capacity and mass transfer performance are important indicators for evaluating adsorbents. Higher adsorption capacity and good mass transfer performance are conducive to obtaining highly pure p-xylene product.

US3960774 and CN1565718A both report a method of treating an adsorbent with an aqueous solution containing sodium hydroxide to convert the binder therein into X zeolite, thereby increasing the adsorption capacity of the adsorbent.

CN1275926A discloses an agglomerated zeolite adsorbent, which uses X zeolite with a Si/Al atomic ratio of 1-1.15 as a raw material and is exchanged with barium and potassium ions. The exchangeable sites in the adsorbent contain at least 70% of barium ions and at most 30% of potassium ions. The adsorbent uses kaolin as a binder and is in-situ crystallized into X zeolite by alkaline solution treatment to increase the adsorption capacity of the adsorbent.

The selectivity of the adsorbent is mainly improved from the aspects of exchange ion types and zeolite properties. US3997620 uses X zeolite adsorbent exchanged with strontium and barium bimetallic ions to show higher p-xylene selectivity.

The prior art also discloses that Y molecular sieve is treated with water vapor to form mesopores and macropores, the purpose of which is to improve the catalytic performance of the molecular sieve, but the adsorption capacity of the molecular sieve cannot be guaranteed.

Therefore, the adsorption capacity and mass transfer performance of the adsorbents obtained in the prior art are still not completely satisfactory. Therefore, the technical problem to be solved by the present invention is how to generate mesopores and macropores while maintaining a high micropore volume, that is, to improve the mass transfer efficiency of adsorptive separation while maintaining a high adsorption capacity of the X-type molecular sieve.

### Summary of the Invention

The first object of the present invention is to provide a modified X-type molecular sieve comprising mesopore and macropore channels, its preparation method and use. The modified X-type molecular sieve exhibits excellent mass transfer performance, adsorption capacity and separation performance of p-xylene in the adsorptive separation of p-xylene.

The second object of the present invention is to provide an adsorbent, in particular an adsorbent for aromatic hydrocarbon isomers, using the modified X-type molecular sieve containing mesopore and macropore channels as an active component, and its preparation method and use. The adsorbent exhibits excellent mass transfer performance, adsorption capacity and separation performance of p-xylene in the adsorptive separation of p-xylene.

In order to achieve the first object of the present invention, in the first aspect, the present invention provides a modified X-type molecular sieve, characterized in that, its surface area as measured by nitrogen physical adsorption method is 9.5-19 m²/g, and the sum of the mesopore and macropore volumes accounts for 6-18% of the total pore volume, wherein the mesopores are pores with a pore diameter of 10nm to 50nm, and the macropores are pores with a pore diameter greater than 50nm and less than 80nm.

In the second aspect, the present invention provides a preparation method of the modified X-type molecular sieve, characterized in that it comprises the following steps:
(1) placing the X-type molecular sieve in a water vapor atmosphere to conduct hydrothermal treatment, preferably at a temperature of 250-550 °C for at least 0.5 hours, then washing and drying it to obtain a first product;
(2) placing the first product obtained at the end of step (1) in an aqueous solution containing NaOH, SiO₂ and an organic ammonium salt, preferably at a temperature of 60-120 °C for at least 0.5 hours, then washing, drying and calcining it to obtain the modified X-type molecular sieve.

In the third aspect, the present invention relates to a use of the modified X-type molecular sieve for an adsorptive separation of aromatic hydrocarbon isomers, especially p-xylene, characterized in that the modified X-type molecular sieve or the modified X-type molecular sieve prepared by the preparation method of the modified X-type molecular sieve is used as an adsorbent.

In order to achieve the above second object of the present invention, in the fourth aspect, the present invention provides an adsorbent for aromatic hydrocarbon isomers, in particular an adsorbent for p-xylene, characterized in that it comprises the modified X-type molecular sieve according to the first aspect, wherein the surface area of the modified X-type molecular sieve is 9.5-19 m²/g, the sum of the mesopore and macropore volumes accounts for 6%-18% of the total pore volume, wherein the mesopores are pores with a pore diameter of 10nm to 50nm, and the macropores are pores with a pore diameter of greater than 50nm and less than 80nm. According to a preferred embodiment, the adsorbent for aromatic hydrocarbon isomers comprises 88-95% by mass, preferably 90-94% by mass of the modified X-type molecular sieve; preferably, the adsorbent for aromatic hydrocarbon isomers also comprises 4-11% by mass of transformed crystal X-type molecular sieve and 1-3% by mass of matrix.

In a fifth aspect, the present invention provides a preparation method of the above-mentioned adsorbent for aromatic hydrocarbon isomers, comprising the following steps:
(1) placing the X-type molecular sieve in a water vapor atmosphere to conduct hydrothermal treatment, then washing and drying it to obtain a first product;
(2) placing the first product obtained in step (1) in an aqueous solution containing NaOH, SiO₂ and an organic ammonium salt to conduct treatment, then washing, drying and calcining it to obtain the modified X-type molecular sieve;
(3) mixing and forming the modified X-type molecular sieve obtained in step (2) with a binder, then calcining it to obtain a formed body;
(4) treating the formed body obtained in step (3) with an alkaline solution to in-situ crystallize the binder therein, and then drying it;
(5) subjecting the product obtained in step (4) to barium ion exchange and optionally potassium ion exchange, and then drying it to obtain the adsorbent for aromatic isomers.

According to a preferred embodiment, the present invention provides a preparation method of the above adsorbent for aromatic hydrocarbon isomers, characterized in that it comprises the following steps:
(1) placing the X-type molecular sieve in a water vapor atmosphere to conduct hydrothermal treatment at a temperature of 250-550 °C for at least 0.5 hours, then washing and drying it to obtain a first product;
(2) placing the first product obtained in step (1) in an aqueous solution containing NaOH, SiO₂ and an organic ammonium salt to conduct treatment at a temperature of 60-120 °C for at least 0.5 hours, then washing, drying and calcining it to obtain the modified X-type molecular sieve;
(3) forming the modified X-type molecular sieve obtained in step (2) with a binder, drying and calcining it to obtain a formed body, wherein the shape of the formed body is preferably a small ball;
(4) treating the formed body obtained at the end of step (3) with a mixed solution of sodium hydroxide and potassium hydroxide to in-situ crystallize the binder therein, and then drying it;
(5) subjecting the dried formed body obtained in step (4) to cation exchange with a solution of a soluble barium salt, or a solution of a soluble barium salt and a soluble potassium salt, then conducting drying and activation.

In the sixth aspect, the present invention also relates to a use of the adsorbent for adsorptive separation of aromatic hydrocarbon isomers, especially p-xylene.

The modified X-type molecular sieve and the adsorbent comprising the modified X-type molecular sieve provided by the present invention maintains a relatively high adsorption capacity, at the same time it has significantly improved mass transfer performance due to containing mesopore and macropore channels, and can improve the separation performance of aromatic hydrocarbon isomers, especially p-xylene.

### Brief Description of Figures

The following figures together with the following specific embodiments are used to explain the present application, but in no way constitute a limitation on the protection scope of the present invention, wherein:

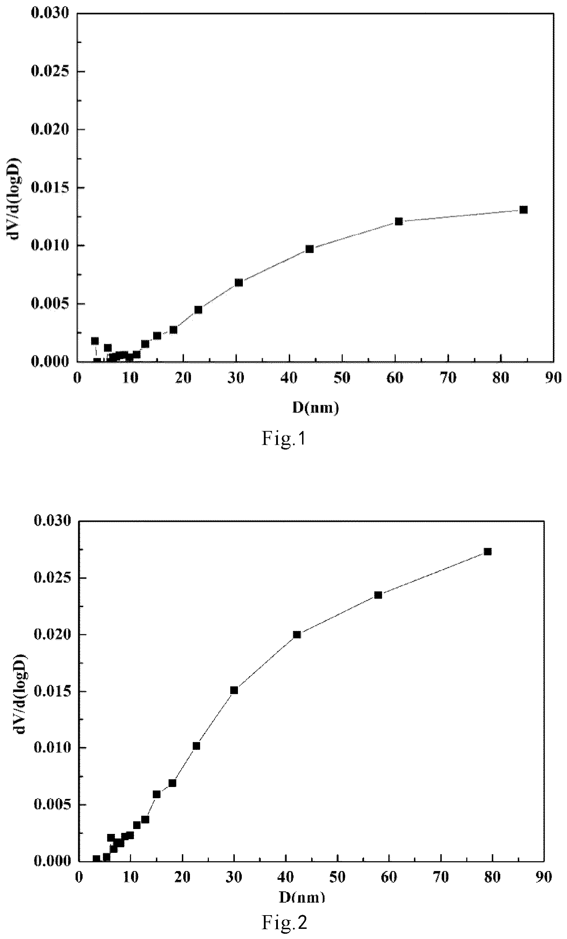

FIG.1 is a pore diameter distribution curve of mesopores and macropores of the X-type molecular sieve used in step (1) of Example 1;
FIG.2 is a pore diameter distribution curve of mesopores and macropores of the modified X-type molecular sieve A obtained in step (2) of Example 1;
FIG.3 is a schematic diagram of the small-scale simulated moving bed adsorptive separation implemented in Example 13.

### Detailed Description of the Invention

The specific embodiments of the present application are described in detail below, but it should be noted that the protection scope of the present application is not limited by these specific embodiments, but is determined by the appendix claims.

Any specific numerical value disclosed herein (including the endpoints of the numerical range) is not limited to the exact value of the numerical value, but should be understood to also cover values close to the exact value, such as all possible values within the range of ±5% of the exact value. In addition, for the disclosed numerical range, one or more new numerical ranges can be obtained by arbitrarily combine the endpoint values of the range, the endpoint value and the specific point value in the range, and the specific point values in the range, and these new numerical ranges should also be regarded as specifically disclosed herein.

Unless otherwise specified, the terms used herein have the same meaning as commonly understood by those skilled in the art. If a term is defined herein and its definition is different from the commonly understood meaning in the art, the definition herein shall prevail.

In the present application, the pore volume and the surface area of the molecular sieve are determined by nitrogen physical adsorption method.

In the present application, except for the contents clearly stated, any matters or items not mentioned are directly applicable to the aspects known in the art without any changes. Moreover, any embodiment described herein can be freely combined with one or more other embodiments described herein, and the technical solutions or technical ideas formed thereby are deemed to be part of the original disclosure or original record of the present application, and should not be regarded as new contents not disclosed or anticipated herein, unless a person skilled in the art considers that such combination is obviously unreasonable.

All patents and non-patent literatures, including but not limited to textbooks and journal articles, cited herein are incorporated herein by reference in their entirety.

The inventor believes that the adsorption capacity and the mesoporous channels are indispensable for maintaining high separation performance of X-type molecular sieve. Without being limited to specific theories, the inventor surprisingly found that by treating the X-type molecular sieve in a water vapor atmosphere at a higher temperature, a small amount of damage is caused to its skeleton structure, and then the X-type molecular sieve whose skeleton structure has been slightly damaged is placed in an alkaline solution containing NaOH, SiO₂ and an organic ammonium salt to conduct treatment, and the partially damaged X-type molecular sieve is repaired, and at the same time, the organic amine substance formed by the organic ammonium salt enters the mesoporous channels to occupy the position, and blocks the crystal growth at this position, thereby forming mesopores and macropore channels while maintaining a high micropore volume. The formation of such mesopores and macropore channels is conducive to improving the mass transfer performance of the X-type molecular sieve, and the higher micropore volume can ensure the adsorption capacity of the X-type molecular sieve, thereby improving the separation performance of aromatic hydrocarbon isomers, especially p-xylene. According to the preparation method of the modified X-type molecular sieve of the present invention, by combining the high-temperature water vapor treatment in step (1) with the modification step of treating with an alkaline aqueous solution containing NaOH, SiO₂ and an organic ammonium salt in step (2), a modified X-type molecular sieve can be obtained, which has a higher adsorption capacity and significantly improved mass transfer performance.

As described above, the present invention provides a modified X-type molecular sieve, whose surface area is 9.5-19m²/g, preferably 10-18m²/g, such as 11m²/g, 12m²/g, 13m²/g, 14m²/g, 15m²/g, 16m²/g or 17m²/g, as measured by nitrogen physical adsorption method; the sum of the mesopore and macropore volumes accounts for 6%-18% of the total pore volume, preferably 8%-15%, more preferably 10.5%-15%; the content of amorphous species is 2-12% by mass, preferably 2.5-10.0% by mass, more preferably 3.0-8.0% by mass, such as 4.0% by mass, 5% by mass, 6% by mass or 7% by mass; wherein the mesopores refer to the pores having a pore diameter of 10nm to 50nm, and the macropores are pores having a pore diameter of greater than 50nm and less than 80nm. Preferably, the modified X-type molecular sieve has a micropore volume of 0.290-0.340 g/ml, preferably 0.300-0.330 g/ml, and a total pore volume of 0.340-0.390 g/ml, preferably 0.350-0.380 g/ml, wherein the micropores refer to the pores with pore diameter less than 2 nm in the X-type molecular sieve.

According to a preferred embodiment, in the modified X-type molecular sieve, the SiO₂/Al₂O₃ molar ratio is 2.21-2.55, such as 2.25, 2.3, 2.4 or 2.5, preferably 2.25-2.4.

According to a preferred embodiment, the particle size of the modified X-type molecular sieve is 0.6 µm-2.5 µm, for example, 0.8 µm, 0.9 µm, 1.0 µm, 1.2 µm, 1.4 µm, 1.6 µm, 1.8 µm, 2.0 µm, 2.2 µm, 2.4 µm or 2.5 µm, preferably 0.8 µm -1.5 µm.

According to a preferred embodiment, the cation contained in the modified X-type molecular sieve is Na, or the cations contained in the modified X-type molecular sieve are Na and K.

The present invention also provides a preparation method of the modified X-type molecular sieve, comprising the following steps:
(1) placing an X-type molecular sieve in a water vapor atmosphere to conduct treatment at a temperature of 250-550°C for at least 0.5 hours, and then washing and drying it to obtain a first product;
(2) placing the first product obtained in step (1) in an aqueous solution containing NaOH, SiO₂ and an organic ammonium salt to conduct treatment at a temperature of 60-120°C for at least 0.5 hours, then washing, drying and calcining it to obtain the modified X-type molecular sieve.

According to a preferred embodiment, in step (1) of the preparation method of the modified X-type molecular sieve, the SiO₂/Al₂O₃ molar ratio of the X-type molecular sieve used is 2.0-2.5, such as 2.2, 2.3 or 2.4, and the particle size thereof is 0.6-2.5 µm, such as 0.8 µm, 0.9 µm, 1.0 µm, 1.2 µm, 1.4 µm, 1.6 µm, 1.8 µm, 2.0 µm, 2.2 µm, 2.4 µm or 2.5 µm. Further preferably, the SiO₂/Al₂O₃ molar ratio of the X-type molecular sieve used is 2.25-2.40, and the particle size is 0.8-1.5 µm.

According to a preferred embodiment, the water vapor atmosphere used in step (1) of the preparation method comprises water vapor and an inert gas, or water vapor and air, wherein the inert gas is preferably nitrogen, carbon dioxide, argon, etc. The temperature of the water vapor atmosphere is preferably 300-500 °C; the water vapor partial pressure in the water vapor atmosphere is preferably 0.6-1.0 bar, such as 0.7 bar, 0.8 bar and 0.9 bar; preferably, the treatment time is at least 1 hour, preferably at least 2 hours, at least 3 hours, such as 4 hours, 5 hours, 6 hours, and at most 50 hours, at most 40 hours, at most 30 hours, preferably at most 20 hours, preferably at most 10 hours.

According to a preferred embodiment, in step (1) of the preparation method of the modified X-type molecular sieve, the X-type molecular sieve is washed with deionized water, preferably, the ratio of the volume of the deionized water used to the volume of the X-type molecular sieve is at least 3:1, preferably 20:1 to 5:1; and the X-type molecular sieve is dried at a temperature of 60-130°C for 1-20 hours, preferably 2-15 hours.

According to a preferred embodiment, in the aqueous solution containing NaOH, SiO₂ and an organic ammonium salt used in step (2) of the preparation method of the modified X-type molecular sieve, the concentration of NaOH is 1.0-2.0 mol/L, preferably 1.2-1.8 mol/L, more preferably 1.4-1.6 mol/L; the concentration of SiO₂ is 5.0-20.0 g/L, preferably 6.0-18.0 g/L, more preferably 8.0-16.0 g/L; the concentration of the organic ammonium salt is 0.0005-0.05 mol/L, such as 0.003 mol/L, 0.005 mol/L, 0.01 mol/L, 0.013 mol/L, 0.018 mol/L, 0.023 mol/L, 0.028 mol/L, 0.033 mol/L, 0.038 mol/L, 0.043 mol/L, 0.048 mol/L, preferably 0.001-0.04 mol/L, more preferably 0.008-0.03 mol/L. The ratio of the aqueous solution containing NaOH, SiO₂ and an organic ammonium salt to the first product is 2-5:1, preferably 3-4:1, wherein the aqueous solution containing NaOH, SiO₂ and an organic ammonium salt is measured in milliliter, and the first product is measured in gram. Preferably, the treatment time of the first product in the aqueous solution containing NaOH, SiO₂ and an organic ammonium salt is at least 1 hour, preferably at least 1.5 hours, preferably at least 3 hours, and at most 50 hours, preferably at most 40 hours, 30 hours, 20 hours or 15 hours, and the treatment temperature is 60-120 °C, preferably 70-100 °C, more preferably 80-90 °C.

According to a preferred embodiment, in step (2) of the preparation method of the modified X-type molecular sieve, the product treated with the above aqueous solution is washed with deionized water until the pH value of the deionized water is less than 10, and dried at a temperature of 60-130°C, preferably 70-120 °C, for 1-20 hours, preferably 2-15 hours.

According to a preferred embodiment, the total carbon number of the organic ammonium salt used in step (2) of the preparation method of the modified X-type molecular sieve is 9-25, preferably 11-20, and preferably contains one or more C3-20 alkyl groups, such as dodecyltrimethylammonium chloride, tetradecyltrimethylammonium chloride, hexadecyltrimethylammonium chloride, octadecyltrimethylammonium chloride, etc.

According to a preferred embodiment, the calcination performed in step (2) of the preparation method of the modified X-type molecular sieve is a multi-stage (n stages) calcination, wherein n ≥ 4, for example, n = 5, 6, 7, etc.; in the multi-stage calcination, the time of constant temperature of each stage is ≥ 1.0 hour, such as 1.5 hours, 2 hours, 3 hours, 4 hours, etc., and the maximum temperature of each stage does not exceed 550°C, for example, 530°C, 500°C, 450°C.

The water vapor treatment carried out in step (1) of the preparation method of the modified X-type molecular sieve produces amorphous species; when treated with an aqueous solution containing NaOH, SiO₂ and an organic ammonium salt in step (2), the amorphous species will not be completely crystallized into the X-type molecular sieve, and a small amount of amorphous species will remain, and the local high temperature generated by the combustion of the organic amine during the calcination process in step (2) partially destroys the molecular sieve structure, and the amorphous species is formed again.

Method for determining the content of amorphous species: the micropore volume of the X type molecular sieve and the micropore volume of the modified X type molecular sieve measured by nitrogen physical adsorption are V₁ and V₂, respectively, and the content of amorphous species is 100% - V₂/V₁.

The present invention also provides an adsorbent for aromatic hydrocarbon isomers, in particular an adsorbent for C8 aromatic hydrocarbon, preferably an adsorbent for p-xylene, which comprises the modified X-type molecular sieve, more preferably 88-95% by mass of the modified X-type molecular sieve, 4-11% by mass of the transformed-crystal X-type molecular sieve and 1-3% by mass of the matrix, and the surface area of the modified X-type molecular sieve is 9.5-19m²/g as measured by nitrogen physical adsorption method, and the sum of the mesopore and macropore volumes accounts for 6%-18% of the total pore volume, wherein the mesopores are pores with a pore diameter of 10nm to 50nm, and the macropores are pores with a pore diameter of greater than 50nm and less than 80nm. The adsorption capacity of the adsorbent for aromatic hydrocarbon isomers as measured by toluene dynamic adsorption method is 175-186 mg/g, preferably 177-184 mg/g.

The transformed-crystal X-type molecular sieve refers to the crystallized molecular sieve portion obtained by in-situ crystallization of a calcined formed body containing a binder and a modified molecular sieve by using an alkaline solution such as a mixed solution of sodium hydroxide and potassium hydroxide during the preparation of the adsorbent.

According to the present application, the matrix may be a binder, for example, may be selected from kaolin, dickite, perlite, refractory stone, halloysite, hydromica, montmorillonite, or a combination thereof.

As the second object of the present invention, the present invention provides a preparation method of the above-mentioned adsorbent for aromatic hydrocarbon isomers, comprising the following steps:
(1) placing the X-type molecular sieve in a water vapor atmosphere to conduct hydrothermal treatment, and then washing and drying it to obtain a first product;
(2) placing the first product obtained in step (1) in an aqueous solution containing NaOH, SiO₂ and an organic ammonium salt to conduct treatment, and then washing, drying and calcining it to obtain the modified X-type molecular sieve;
(3) mixing and forming the modified X-type molecular sieve obtained in step (2) with a binder, and calcining it to obtain a formed body;
(4) treating the formed body obtained in step (3) with an alkaline solution to in-situ crystallize the binder therein, and then drying it;
(5) subjecting the product obtained in step (4) to barium ion exchange and optionally potassium ion exchange, and then drying it to obtain the adsorbent for aromatic isomer.

In step (3) of the preparation method of the adsorbent, the forming can be achieved by various forming methods commonly used in the art, and the present application does not impose strict restrictions on this.

In particular, the present invention also provides a preparation method of the adsorbent for aromatic hydrocarbon isomers, in particular the adsorbent for C8 aromatic hydrocarbon, preferably the adsorbent for p-xylene, characterized in that it comprises the following steps:
(1) placing the X-type molecular sieve in a water vapor atmosphere to conduct hydrothermal treatment at a temperature of 250-550 °C for at least 0.5 hours, and then washing and drying to obtain a first product;
(2) placing the first product obtained in step (1) in an aqueous solution containing NaOH, SiO₂ and an organic ammonium salt to conduct treatment at a temperature of 60-120°C for at least 0.5 hours, then washing, drying and calcining to obtain the modified X-type molecular sieve;
(3) uniformly mixing the modified X-type molecular sieve obtained in step (2) with a binder to perform forming, then drying and calcining it to obtain a formed body, wherein the formed body is preferably in the shape of a small ball;
(4) treating the formed body obtained at the end of step (3) with a mixed solution of sodium hydroxide and potassium hydroxide to in-situ crystallize the binder therein, and then drying it;
(5) subjecting the dried formed body obtained in step (4) to cation exchange with a solution of a soluble barium salt or a solution of soluble barium and potassium salts, and then drying and activating it.

According to a preferred embodiment, the SiO₂/Al₂O₃ molar ratio of the X-type molecular sieve used in step (1) of the preparation method of the adsorbent is 2.0-2.5, such as 2.2, 2.3 or 2.4, and the particle size of the X-type molecular sieve is 0.6-2.5 µm, such as 0.8 µm, 0.9 µm, 1.0 µm, 1.2 µm, 1.4 µm, 1.6 µm, 1.8 µm, 2.0 µm, 2.2 µm, 2.4 µm or 2.5 µm. Further preferably, the SiO₂/Al₂O₃ molar ratio of the X-type molecular sieve used is 2.25-2.40, and the particle size is 0.8-1.5 µm.

According to a preferred embodiment, the water vapor atmosphere used in step (1) of the preparation method of the adsorbent comprises water vapor and an inert gas or air. The inert gas is preferably nitrogen, carbon dioxide, argon, etc. The water vapor partial pressure in the water vapor atmosphere is preferably 0.6-1.0 bar, such as 0.7 bar, 0.8 bar and 0.9 bar; preferably, the treatment time is at least 1 hour, preferably at least 2 hours, at least 3 hours, such as 4 hours, 5 hours, 6 hours, and at most 50 hours, at most 40 hours, at most 30 hours, preferably at most 20 hours, preferably at most 10 hours.

According to a preferred embodiment, in step (1) of the preparation method of the adsorbent, the X-type molecular sieve is washed with deionized water, preferably, the ratio of the volume of the deionized water used to the volume of the X-type molecular sieve is at least 3:1, preferably 20:1 to 5:1; and it is dried at a temperature of 60-130°C for 1-20 hours, preferably 2-15 hours.

According to a preferred embodiment, in the aqueous solution containing NaOH, SiO₂ and an organic ammonium salt used in step (2) of the preparation method of the adsorbent, the concentration of NaOH is 1.0-2.0 mol/L, preferably 1.2-1.8 mol/L, more preferably 1.4-1.6 mol/L; the concentration of SiO₂ is 5.0-20.0 g/L, preferably 6-18 g/L, more preferably 8-16 g/L; the concentration of the organic ammonium salt is 0.0005-0.05 mol/L, for example 0.001 mol/L, 0.003 mol/L, 0.005 mol/L, 0.01 mol/L, 0.013 mol/L, 0.018 mol/L, 0.023 mol/L, 0.028 mol/L, 0.033 mol/L, 0.038 mol/L, 0.043 mol/L, 0.048 mol/L, preferably 0.001-0.04 mol/L, more preferably 0.01-0.03 mol/L. The ratio of the aqueous solution containing NaOH, SiO₂ and an organic ammonium salt to the first product is 2-5:1, such as 3:1 or 4:1, wherein the aqueous solution containing NaOH, SiO₂ and an organic ammonium salt is measured in milliliter, and the first product is measured in gram. Preferably, the treatment time of the first product in the aqueous solution containing NaOH, SiO₂ and an organic ammonium salt is at least 1 hour, preferably at least 1.5 hours, preferably at least 3 hours, and at most 50 hours, preferably at most 40 hours, 30 hours, 20 hours or 15 hours.

According to a preferred embodiment, in step (2) of the preparation method of the adsorbent, the treated product is washed with deionized water until the pH value of the deionized water is less than 10, and dried at a temperature of 60-130°C for 1-20 hours, preferably 2-15 hours.

According to a preferred embodiment, the total carbon number of the organic ammonium salt used in step (2) of the preparation method of the adsorbent is 9-25, preferably 11-20, and preferably contains one or more C3-20 alkyl groups, such as dodecyltrimethylammonium chloride, tetradecyltrimethylammonium chloride, hexadecyltrimethylammonium chloride, octadecyltrimethylammonium chloride, etc.

According to a preferred embodiment, the calcination performed in step (2) of the preparation method of the adsorbent is a multi-stage calcination, and preferably the number of calcination stages n is ≥ 4, for example n = 5, 6, 7, etc.; in the multi-stage calcination, the time of constant temperature of each stage is ≥ 1.0 hour, for example 1.5 hours, 2 hours, 3 hours or 4 hours, etc., and the maximum temperature of each stage does not exceed 550°C, for example 530°C, 500°C or 400°C.

According to a preferred embodiment, in step (3) of the preparation method of the adsorbent, the mass ratio of the modified X-type molecular sieve to the binder is 88-95:5-12, for example 89:11, 90:10, 91:9, 92:8, 93:7 or 94:6.

According to a preferred embodiment, in step (3) of the preparation method of the adsorbent, the forming is ball-rolling, which includes gradually aggregating and growing the forming powder obtained by mixing the X-type molecular sieve and the binder in a rotating disk, and continuously spraying water during the ball-rolling to moisten the surface of the small ball and adhere the powder.

According to a preferred embodiment, in step (3) of the preparation method of the adsorbent, the forming is ball-rolling, wherein the amount of water added for rolling into balls is 6-22% by mass of the total amount of solid powder, for example, 7%, 8%, 9%, 10%, 11%, 12%, 13%, 16%, 17%, 18%, 19% and 20% by mass.

According to a preferred embodiment, in step (3) of the preparation method of the adsorbent, the binder used is selected from kaolin, dickite, perlite, refractory stone, halloysite, hydromica, montmorillonite, or a combination thereof. Further preferably, the binder used contains 75-95% by mass of kaolin and 5-15% by mass of halloysite. The binder with this composition can be better transformed into an X-type molecular sieve during the in-situ crystallization in step (4), thereby improving the adsorption capacity and separation performance of the adsorbent.

According to a preferred embodiment, in step (3) of the preparation method of the adsorbent, the drying temperature is 80-150°C, for example, 90°C, 100°C, 110°C, 120°C, 130°C or 140°C; the drying time is 0.5-20.0 hours, for example, 1.0 hour, 2.0 hours, 3.0 hours, 4.0 hours, 5.0 hours, 6.0 hours, 9.0 hours or 12.0 hours.

According to a preferred embodiment, in step (3) of the preparation method of the adsorbent, the calcination temperature is 500-700°C, for example, 500°C, 520°C, 540°C, 560°C, 580°C, 600°C, 620°C, 640°C, 660°C, 680°C or 700°C; the calcination time is 0.5-6.0 hours, for example, 0.5 hours, 1.0 hour, 1.5 hours, 2.0 hours, 2.5 hours, 3.0 hours, 3.5 hours, 4.0 hours, 4.5 hours, 5.0 hours, 5.5 hours or 6.0 hours.

According to a preferred embodiment, in step (4) of the preparation method of the adsorbent, the in-situ crystallization treatment temperature is 80-100°C, for example, 85°C, 90°C, 95°C, and the in-situ crystallization treatment time is 1.5-6.0 hours, for example, 1.5 hours, 2.0 hours, 2.5 hours, 3.0 hours, 3.5 hours, 4.0 hours, 4.5 hours, 5.0 hours, 5.5 hour or 6.0 hours.

According to a preferred embodiment, in the alkaline solution treatment in step (4) of the preparation method of the adsorbent, the liquid-to-solid volume ratio of the alkaline solution to the small balls is 1.2-4.0:1, for example, 1.2:1, 1.4:1, 1.6:1, 1.8:1, 2.0:1, 2.2:1, 2.4:1, 2.6:1, 2.8:1 or 3.0:1, preferably 2.0-3.0:1.

According to a preferred embodiment, in step (4) of the preparation method of the adsorbent, the hydroxide ion concentration in the mixed solution is 0.1-2.0 mol/L, preferably 0.3-1.8 mol/L, more preferably 0.5-1.5 mol/L; the K/(Na+K) molar ratio is 0.1-0.6, for example 0.2, 0.3, 0.4, 0.5.

In step (5) of the preparation method of the adsorbent for p-xylene, the small balls obtained in step (4) are subjected to cation exchange with a mixed solution of a soluble barium salt and a soluble potassium salt; or the small balls obtained in step (4) are subjected to cation exchange with a solution of a soluble barium salt or a soluble potassium salt, respectively, so that at least part of the cation sites of the X-type molecular sieve contained therein are occupied by Ba²⁺ and/or K⁺. Preferably, the ratio of the total number of moles of cations (Ba²⁺ and/or K⁺) in the exchange solution used to the number of moles of Na⁺ in the molecular sieve, i.e., the exchange ratio, is 1.5-3.0:1.

Without being limited to a specific theory, the applicant believes that the cation exchange treatment in step (5) can cause the barium ions, or the barium ions and potassium ions to coordinate with the oxygen ions in the framework of the modified X-type molecular sieve, so that a high PX selective adsorption force field with a symmetry of D₂ group is generated in the modified X-type molecular sieve supercage, and so that the obtained adsorbent has higher separation performance.

According to a preferred embodiment, only barium ion exchange is performed in step (5), and the barium ion exchange is performed by using a solution of a soluble barium salt, and the soluble barium salt is preferably selected from barium chloride, barium nitrate, or a combination thereof.

According to a preferred embodiment, in step (5), the barium ion exchange is performed first and then the potassium ion exchange is performed, or the potassium ion exchange is performed first and then barium ion exchange is performed. The barium ion exchange is performed by using a solution of a soluble barium salt, and the soluble barium salt is preferably selected from barium chloride, barium nitrate, or a combination thereof; the potassium ion exchange is performed by using a soluble potassium salt solution, and the soluble potassium salt is preferably selected from potassium chloride, potassium nitrate, or a combination thereof.

According to a preferred embodiment, in step (5), the barium ion and potassium ion exchange are simultaneously carried out by using a mixed solution containing a soluble barium salt and a soluble potassium salt, and the soluble barium salt and the soluble potassium salt are selected as described above.

According to a preferred embodiment, the conditions for the barium ion exchange and the optional potassium ion exchange in step (5) include: the temperature being 60-97°C, for example, 65°C, 70°C, 75°C, 80°C, 85°C, 90°C or 95°C, preferably 80-95°C, and the time being 2.0-48.0 hours, for example, 2.0 hours, 6.0 hours, 10.0 hours, 14.0 hours, 18.0 hours, 22.0 hours, 26.0 hours, 30.0 hours, 34.0 hours, 38.0 hours, 42.0 hours, 46.0 hours, 48.0 hours, preferably 12.0-36.0 hours.

According to a preferred embodiment, the volume space velocity of the solution of barium salt, the solution of potassium salt, and the mixed solution of barium salt and potassium salt in the ion exchange in step (5) is independently 1.0-20.0 h⁻¹, for example, 1.0 h⁻¹, 2.0 h⁻¹, 3.0 h⁻¹, 4.0 h⁻¹, 5.0 h⁻¹, 6.0 h⁻¹, 7.0 h⁻¹, 8.0 h⁻¹, 9.0 h⁻¹, 10.0 h⁻¹, 12.0 h⁻¹, 14.0 h⁻¹, 16.0 h⁻¹, 18.0 h⁻¹ or 20.0 h⁻¹, preferably 1.0-10.0 h⁻¹.

According to a preferred embodiment, the product obtained after ion exchange in step (5) is dried to obtain an adsorbent based on the modified X-type molecular sieve, the drying temperature is preferably 60-130°C, preferably 70-120°C, and the drying time is 1.0-24.0 hours, preferably 3-15 hours.

According to a preferred embodiment, the cation exchanged small balls in step (5) are washed with deionized water, wherein the ratio of the volume of deionized water to the volume of the molecular sieve is 3-20, preferably 5-15, and more preferably 6-12.

According to a preferred embodiment, the activation in step (5) is carried out in a nitrogen atmosphere at 50-120°C, preferably 60-100°C, for 5-30 hours, preferably 10-24 hours.

The present invention is further described below by way of examples, but the present invention is not limited thereto.

The surface area and pore volume of the X-type molecular sieve sample were measured by using an ASAP 2020 Physical Adsorption Apparatus from Micromeritics, USA. Before the measurement, the X-type molecular sieve sample was degassed at 300-450°C and vacuum (<10⁻² Pa) for 6-16 hours, preferably degassed at 300°C for 10.0 hours, and nitrogen physical adsorption was performed at 77K. The micropore volume V₁ of the X-type molecular sieve sample was determined by using the t-plot method. The sum of the mesopore and macropore volumes was the difference between the total pore volume V at P/P₀ =0.99 minus the micropore volume V₁, wherein P is the pressure during the measurement and P₀ is 0.1MPa.

The elemental chemical composition of the molecular sieve is characterized by X-ray fluorescence spectrometry (XRF), and the instrument used is Shimadzu XRF-1800 X-ray Fluorescence Spectrometer. The qualitative analysis of BaO, K₂O, Na₂O, Al₂O₃ and SiO₂ is carried out according to the corresponding relationship between the wavelength of characteristic X-ray fluorescence and the atomic number. The quantitative analysis of BaO, K₂O, Na₂O, Al₂O₃ and SiO₂ can be carried out according to the comparison of the peak intensity at the wavelength of X-ray fluorescence with that of the standard sample. The SiO₂/Al₂O₃ molar ratio of the molecular sieve is calculated by the relative mass of SiO₂ and Al₂O₃.

The information of the molecular sieve crystal size is characterized by FE-SEM photos. The instrument used is a Japanese Hitachi S-4800 Scanning Electron Microscope. The crystal size within the range of 10.0 µm × 10.0 µm is selected for measurement and calculation. The measured crystal size is the average value of all crystal sizes.

The dynamic pulse test device is used to measure the separation performance of the X-type molecular sieve and the separation performance of the adsorbent containing the X-type molecular sieve.

The method for determining the adsorption capacity of the adsorbent is: contacting nitrogen gas carrying toluene (toluene partial pressure is 0.05 MPa) with a certain mass of adsorbent at 35°C until toluene reaches adsorption equilibrium. The adsorption capacity of the tested adsorbent material is calculated based on the mass difference of the adsorbent before and after toluene adsorption. Since the kinetic diameters of toluene and p-xylene are close and both can be adsorbed in the micropore channels of the X-type molecular sieve, so the adsorption capacity of p-xylene in the X-type molecular sieve is positively correlated with the adsorption capacity measured by toluene.

The dynamic pulse test device is composed of a feed system, an adsorption column, a heating furnace, a pressure control valve, etc. The adsorption column is a stainless steel tube of Φ6× 1800 mm, and the adsorption material loading is 50 ml. The lower inlet of the adsorption column is connected to the system of feed and nitrogen, and the upper outlet is connected to the pressure control valve, and then connected to the effluent collector. The desorbent used in the experiment is 30% by volume of p-diethylbenzene (PDEB) and 70% by volume of n-heptane. The pulse feed liquid is composed of 5% by volume of ethylbenzene (EB), p-xylene (PX), m-xylene (MX), o-xylene (OX), n-nonane (NC9) and 75% by volume of desorbent.

Before the dynamic pulse test evaluation, the X-type molecular sieve sample needs to be ion exchanged, wherein the specific steps are: the powdered molecular sieve is pressed into tablets under a pressure of 25MPa, and then crushed and sieved to obtain particles of 300-850µm. A mixed solution of 0.18mol/L barium chloride and 0.07mol/L potassium chloride is used to conduct ion exchange; the ion exchange is carried out in a column container at an exchange temperature of 90°C for 8 hours; the liquid volume space velocity of the ion exchange is 6 h⁻¹.

The method for determining adsorption selectivity is: loading the weighed adsorbent to be measured having a particle size of 300-850µm into the adsorption column and shaking it, dehydrating and activating it at 160-190°C in a nitrogen atmosphere; then introducing a desorbent to remove the gas in the system; raising the system pressure to 0.8MPa, raising the temperature to 177°C, stopping feeding the desorbent, and feeding 8 ml of pulse feed liquid at a volume space velocity of 1.0 h⁻¹, then feeding the desorbent at a volume space velocity of 1.5 h⁻¹, taking 3 drops of desorbent sample every 2 minutes, and conducting analysis by gas chromatography; the desorption curves of the above components are plotted with the volume of desorbent for desorption as the horizontal axis and the concentration of each component NC9, EB, PX, MX and OX as the vertical axis, wherein NC9 is not adsorbed and can be used as a tracer to obtain the dead volume of the adsorption system; taking the midpoint of the half-peak width of the tracer as the zero point, and measuring the net retention volume R from the midpoint of the half-peak width of each component EB, PX, MX, and OX to the zero point. The net retention volume of any component is proportional to the distribution coefficient at the adsorption equilibrium, reflecting the interaction between each component and the adsorbent material. The ratio of the net retention volume of the two components is the selectivity β. For example, the ratio of the net retention volume of PX to the net retention volume of EB is the ratio of the adsorption performance of the adsorbent material for PX and EB, which is the adsorption selectivity of PX relative to EB, recorded as β_{P/E}. The half-peak width W_{1/2} of PX represents the mass transfer performance; the smaller W_{1/2} is, the better the mass transfer performance is.

The adsorbent prepared by the present invention is suitable for the liquid phase adsorptive separation process of aromatic hydrocarbon isomers, especially the adsorptive separation of p-xylene from a mixture of o-xylene, m-xylene, p-xylene and ethylbenzene. The liquid phase adsorptive separation can be carried out using a multi-column series mode, or using a simulated moving bed realized by a rotary valve or a solenoid valve group. The operating pressure of the adsorptive separation is 0.3-1.5MPa, and the operating temperature is 120-180°C.

The present invention is further described below by way of examples, but the present invention is not limited thereto. In the following examples and comparative examples, unless otherwise specified, all reagents and raw materials used are commercially available products.

### Example 1

Preparation of the modified X-type molecular sieve:
(1) 100 g of X-type molecular sieve (having a SiO₂/Al₂O₃ molar ratio of 2.43, a particle size of 1.0 µm, a micropore volume of 0.335 g/mL, a total pore volume of 0.351 g/mL and a surface area of 5.5 m²/g, the sum of mesopore and macropore volumes accounting for 4.6% of total pore volume, and the pore diameter distribution being shown in Figure (1)) was placed in a water vapor atmosphere of a mixture of water vapor and air at a water vapor partial pressure of 0.7 bar, treated at 300°C for 5 hours, then washed with deionized water and dried at 110°C for 5 hours to obtain a first product;
(2) 60 g of the first product obtained in step (1) was placed in 200 mL of an alkaline solution having a NaOH concentration of 1.2 mol/L, a SiO₂ concentration of 8.0 g/L, and a hexadecyltrimethylammonium chloride concentration of 0.003 mol/L, and treated at 95°C for 3.0 hours, washed with deionized water until the pH value was less than 10, dried at 100°C for 5 hours, and then calcined at a constant temperature of 200°C for 1.5 hours, at a constant temperature of 300°C for 1.5 hours, at a constant temperature of 400°C for 1.5 hours, and at a constant temperature of 550°C for 3.0 hours to obtain a modified X-type molecular sieve sample A.

The surface area of the modified X-type molecular sieve sample A measured by nitrogen physical adsorption was 10.3m²/g, the micropore volume was 0.324 g/mL, the total pore volume was 0.355 g/mL, the sum of the mesopore and macropore volumes accounted for 8.7% of the total pore volume, and the amorphous species content was 3.3% by mass. The pore diameter distribution is shown in Figure (2).

The modified X-type molecular sieve A was pressed into tablets under a pressure of 25MPa; and after being crushed and sieved, particles of 300-850µm were obtained. Ion exchange was carried out in a column container by using a mixed solution of 0.18mol/L barium chloride and 0.07mol/L potassium chloride; the exchange temperature was 90°C, and the ion exchange was carried out in a column container for 8 hours, the liquid volume space velocity of the ion exchange was 6 h⁻¹. Then vacuum filtration was performed and the obtained product was washed with 2000mL deionized water, and dried at 90°C for 12.0 hours. The adsorption selectivity and PX half-peak width of the modified X-type molecular sieve sample A were evaluated using a pulse test. The results are shown in Table 1.

### Example 2

The preparation and evaluation were performed according to the method of Example 1, except that in step (1), the X type molecular sieve was placed in a water vapor atmosphere of a mixture of water vapor and nitrogen at a water vapor partial pressure of 0.9 bar and treated at a temperature of 500°C for 2 hours to obtain a modified X-type molecular sieve sample B.

The surface area of the modified X-type molecular sieve sample B measured by nitrogen physical adsorption was 10.4m²/g, the micropore volume was 0.307 g/mL, the total pore volume was 0.343 g/mL, the sum of the mesopore and macropore volumes accounted for 10.5% of the total pore volume, and the amorphous species content was 8.4% by mass.

The adsorption selectivity and PX half-peak width are shown in Table 1.

### Example 3-1

The preparation and evaluation were performed according to the method of Example 1, except that in step (2), an alkaline solution having a NaOH concentration of 1.6 mol/L, a SiO₂ concentration of 12.0 g/L, and a dodecyltrimethylammonium chloride concentration of 0.01 mol/L was used to treat the first product obtained in step (1). A modified X-type molecular sieve sample C-1 was obtained.

The surface area of the modified X-type molecular sieve sample C-1 measured by nitrogen physical adsorption was 12.6 m²/g, the micropore volume was 0.327 g/mL, the total pore volume was 0.365 g/mL, the sum of the mesopore and macropore volumes accounted for 10.4% of the total pore volume, and the amorphous species content was 2.4% by mass.

The adsorption selectivity and PX half-peak width are shown in Table 1.

### Example 3-2

The preparation and evaluation were performed according to the method of Example 1, except that in step (2), an alkaline solution having a NaOH concentration of 1.6 mol/L, a SiO₂ concentration of 12.0 g/L, and a dodecyltrimethylammonium chloride concentration of 0.03 mol/L was used to treat the first product obtained in step (1). A modified X-type molecular sieve sample C-2 was obtained.

The surface area of the modified X-type molecular sieve sample C-2 measured by nitrogen physical adsorption was 13.2 m²/g, the micropore volume was 0.322 g/mL, the total pore volume was 0.375g/mL, the sum of the mesopore and macropore volumes accounted for 14.1% of the total pore volume, and the amorphous species content was 2.6% by mass.

The adsorption selectivity and PX half-peak width are shown in Table 1.

### Comparative Example 3-3

The preparation and evaluation were performed according to the method of Example 1, except that in step (2), an alkaline solution having a NaOH concentration of 1.6 mol/L, a SiO₂ concentration of 12.0 g/L, and a dodecyltrimethylammonium chloride concentration of 0 mol/L was used to treat the first product obtained in step (1). A modified X-type molecular sieve sample C-3 was obtained.

The surface area of the modified X-type molecular sieve sample C-3 measured by nitrogen physical adsorption was 5.8 m²/g, the micropore volume was 0.325g/mL, the total pore volume was 0.344g/mL, the sum of the mesopore and macropore volumes accounted for 5.5 % of the total pore volume, and the amorphous species content was 3.0% by mass.

The adsorption selectivity and PX half-peak width are shown in Table 1.

### Example 4

The preparation and evaluation were performed according to the method of Example 1, except that in step (2), after drying at 100°C for 5 hours, and then calcining at a constant temperature of 150°C for 1.0 hours, at 260°C for 1.0 hours, at 380°C for 1.0 hours, at 450°C for 1.0 hours, and at 550°C for 4.0 hours, a modified X-type molecular sieve sample D was obtained.

The surface area of the modified X-type molecular sieve sample D measured by nitrogen physical adsorption was 16.8m²/g, the micropore volume was 0.325 g/mL, the total pore volume was 0.377 g/mL, the sum of the mesopore and macropore volumes accounted for 13.9% of the total pore volume, and the amorphous species content was 3.0% by mass.

The adsorption selectivity and PX half-peak width are shown in Table 1.

### Example 5

The preparation and evaluation were performed according to the method of Example 1, except that in step (2), 400 mL of an alkaline solution having a NaOH concentration of 1.2 mol/L, a SiO₂ concentration of 8.0 g/L, and a hexadecyltrimethylammonium chloride concentration of 0.003 mol/L was used to treat the first product obtained in step (1). A modified X-type molecular sieve sample E was obtained.

The surface area of the modified X-type molecular sieve sample E measured by nitrogen physical adsorption was 11.2 m²/g, the micropore volume was 0.320 g/mL, the total pore volume was 0.354 g/mL, the sum of the mesopore and macropore volumes accounted for 9.6% of the total pore volume, and the amorphous species content was 4.5% by mass.

The adsorption selectivity and PX half-peak width are shown in Table 1.

### Example 6

The preparation and evaluation were performed according to the method of Example 1, except that in step (2), after calcining at a constant temperature of 200°C for 1.5 hours, at 300°C for 1.5 hours, and at 550°C for 3.0 hours, a modified X-type molecular sieve sample F was obtained.

The surface area of the modified X-type molecular sieve sample F measured by nitrogen physical adsorption was 15.6 m²/g, the micropore volume was 0.307 g/mL, the total pore volume was 0.360 g/mL, the sum of the mesopore and macropore volumes accounted for 14.7% of the total pore volume, and the amorphous species content was 8.3% by mass.

The adsorption selectivity and PX half-peak width are shown in Table 1.

### Comparative Example 1

100 g of X-type molecular sieve (having a SiO₂/Al₂O₃ molar ratio of 2.43, a particle size of 1.0 µm, a micropore volume of 0.335 g/mL, a total pore volume of 0.351 g/mL, and a surface area of 5.5 m²/g, the sum of the mesopore and macropore volumes accounting for 4.6% of the total pore volume) was placed in a water vapor atmosphere of a mixture of water vapor and air at a water vapor partial pressure of 0.7 bar, treated at a temperature of 300°C for 5 hours, then washed with deionized water, and dried at a temperature of 110°C for 5 hours to obtain a modified X-type molecular sieve comparative sample DB1.

The surface area of the modified X-type molecular sieve comparative sample DB1 measured by nitrogen physical adsorption was 25.4m²/g, the micropore volume was 0.256g/ml, the total pore volume was 0.306g/ml, the sum of the mesopore and macropore volumes accounts for 16.3% of the total pore volume, and the amorphous species content was 23.5% by mass.

The adsorption selectivity and PX half-peak width are shown in Table 1.

### Comparative Example 2

100 g of X-type molecular sieve (having a SiO₂/Al₂O₃ molar ratio of 2.43, a particle size of 1.0 µm, a micropore volume of 0.335 g/mL, a total pore volume of 0.351 g/mL, and a surface area of 5.5 m²/g, the sum of mesopore and macropore volumes accounting for 4.6% of total pore volume) was placed in 200 mL of alkaline solution with NaOH concentration of 1.2 mol/L, SiO₂ concentration of 8.0 g/L, and hexadecyltrimethylammonium chloride concentration of 0.003 mol/L to contact treatment at 95°C for 3.0 hours, washed with deionized water until the pH value was less than 10, then dried at 100°C for 5 hours, and then calcined at a constant temperature of 200°C for 1.5 hours, 300°C for 1.5 hours, 400°C for 1.5 hours, and 550°C for 3.0 hours to obtain the modified X-type molecular sieve comparative sample DB2.

The surface area of the modified X-type molecular sieve comparative sample DB2 measured by nitrogen physical adsorption was 8.6m²/g, the micropore volume was 0.328 g/mL, the total pore volume was 0.347 g/mL, the sum of the mesopore and macropore volumes accounts for 5.5% of the total pore volume, and the amorphous species content was 2.1% by mass.

The adsorption selectivity and PX half-peak width are shown in Table 1.

### Comparative Example 3

The adsorption selectivity and PX half-peak width of X type molecular sieve (having a SiO₂/Al₂O₃ molar ratio of 2.43, a particle size of 1.0 µm, a micropore volume of 0.335 g/mL, a total pore volume of 0.351 g/mL, and a surface area of 5.5 m ²/g, the sum of the mesopore and macropore volumes accounting for 4.6% of the total pore volume) are shown in Table 1.

**Table 1**

| Example | Molecular sieve samples | Adsorption selectivity | | | W_{1/2}, mL |
|---|---|---|---|---|---|
| | | β_{P/E} | β_{P/M} | β_{P/O} | |
| Example 1 | A | 1.74 | 4.43 | 3.94 | 12.54 |
| Example 2 | B | 1.72 | 4.52 | 4.17 | 11.11 |
| Example 3-1 | C-1 | 1.72 | 4.69 | 4.25 | 11.52 |
| Example 3-2 | C-2 | 1.73 | 4.62 | 4.21 | 11.96 |
| Comparative Example 3-3 | C-3 | 1.72 | 4.25 | 3.46 | 14.19 |
| Example 4 | D | 1.74 | 4.55 | 4.20 | 12.61 |
| Example 5 | E | 1.74 | 4.50 | 4.10 | 12.02 |
| Example 6 | F | 1.72 | 4.26 | 3.88 | 12.93 |
| Comparative Example 1 | DB1 | 1.42 | 2.86 | 2.03 | 16.95 |
| Comparative Example 2 | DB2 | 1.71 | 4.12 | 3.25 | 14.70 |
| Comparative Example 3 | X-type molecular sieve | 1.72 | 4.22 | 3.45 | 14.52 |

In Table 1, W_{1/2} is a direct reflection of the mass transfer performance. The smaller W_{1/2} is, the better the mass transfer performance is. Therefore, the mass transfer performance of Examples 1 to 6 was significantly better than that of Comparative Examples 1 to 4. Example 6 uses three-stage calcination, which makes the rate of temperature increase is too fast compared with the four or five-stage calcination in Examples 1 to 5, then the local high temperature formed by the combustion of the organic amine formed by the organic ammonium salt will destroy the X-type molecular sieve, resulting in a decrease in adsorption capacity and mass transfer performance, and the adsorption selectivity will also decrease accordingly. The adsorption capacity was related to the crystallinity of the X-type molecular sieve.

In Table 1, the adsorption selectivity is affected by the combined effects of mass transfer performance and the adsorption capacity. Comparative Example 1 is treated with water vapor only, the X-type molecular sieve is damaged, the crystallinity is reduced, and a large number of amorphous species are formed. These amorphous species will not only block the mesopores, but also cause the adsorption capacity to be significantly reduced. Therefore, its adsorption selectivity is the worst. Comparative Example 2 uses only alkaline solution to treat the X-type molecular sieve without water vapor treatment. The ratio of the sum of the mesopore and macropore volumes to the total pore volume increases less than that of unmodified X-type molecular sieves, but the calcination process causes the X-type molecular sieve to be slightly damaged, resulting in a decrease in adsorption capacity and mass transfer performance, and the adsorption selectivity also decreases accordingly. Comparative Example 3 is an unmodified X-type molecular sieve, due to the small number of mesopores and macropore structures thereof, the mass transfer performance of the prepared adsorbent is low, and the adsorption selectivity is also low. By comparing Examples 1, 3-1, 3-2 and Comparative Example 3-3, it can be seen that when an alkaline solution is used to treat the X-type molecular sieve, the presence of a relatively low content of an organic ammonium salt in the alkaline solution was necessary and important. The organic ammonium salt can increase the ratio of mesopores and macropores volume, thereby significantly improving the mass transfer performance of the modified X-type molecular sieve. However, an excessively high content of the organic ammonium salt in the alkaline solution has a negative impact on the mass transfer performance and adsorption capacity of the modified X-type molecular sieve.

### Example 7

Preparation of adsorbents containing modified X-type molecular sieves:
(1) 100 kg of X-type molecular sieve (having a SiO₂/Al₂O₃ molar ratio of 2.43, a particle size of 1.0 µm, a micropore volume of 0.335 g/mL, a total pore volume of 0.351 g/mL, and a surface area of 5.5 m²/g, the sum of mesopore and macropore volumes accounting for 4.6% of the total pore volume) was placed in a water vapor atmosphere of a mixture of water vapor and air at a water vapor partial pressure of 0.7 bar, and treated at a temperature of 300°C for 5 hours to obtain a first product;
(2) 98 kg of the first product obtained in step (1) was placed in 300 L of an alkaline solution having a NaOH concentration of 1.2 mol/L, a SiO₂ concentration of 8.0 g/L, and a hexadecyltrimethylammonium chloride concentration of 0.003 mol/L, and treated at a temperature of 95°C for 3.0 hours, washed with deionized water until the pH value was less than 10, dried at 100°C for 5 hours, and then calcined at a constant temperature of 200°C for 1.5 hours, 300°C for 1.5 hours, 400°C for 1.5 hours, and 550°C for 3.0 hours to obtain a modified X-type molecular sieve sample g;
(3) 92 kg of modified X-type molecular sieve and 8 kg of kaolin were mixed evenly, put into a rotating disk to conducting rolling and water was sprayed at the same time to aggregate the solid into small balls, and small balls with a particle size of 300-850 µm were taken, dried at 80°C for 10 hours, and calcined at 540°C for 4 hours to obtain adsorbent matrix small balls;
(4) the small balls obtained in step (3) were subjected to in-situ crystallization treatment using a mixed solution of sodium hydroxide and potassium hydroxide, wherein the hydroxide ion concentration in the mixed solution was 1.0 mol/L, the K/(Na+K) molar ratio was 0.2, and the liquid/solid ratio was 3.0 L/kg. After treatment at 95°C for 4 hours, the small balls were washed with deionized water until the pH value was less than 10, and dried at 100°C for 8 hours;
(5) 130 mL of the small balls obtained in step (4) were loaded into an ion exchange column to conduct cation exchange, and a mixed solution of 0.18 mol/L barium nitrate and 0.07 mol/L potassium nitrate was used for continuous exchange at a volume space velocity of 8.0 h⁻¹ under 0.1 MPa and 94°C for 6 hours, wherein the total amount of the mixed solution was 5000 mL. After the ion exchange was completed, the solid was washed with 700 mL of deionized water at 70°C and activated in a nitrogen atmosphere at 70°C for 24 hours to obtain adsorbent G.

The surface area of the modified X-type molecular sieve sample g measured by nitrogen physical adsorption was 10.3 m²/g, the micropore volume was 0.324 g/mL, the total pore volume was 0.355 g/mL, and the sum of the mesopore and macropore volumes accounted for 8.7% of the total pore volume.

The adsorption selectivity, PX half-peak width and toluene adsorption capacity of adsorbent G were evaluated using a pulse test. The results are shown in Table 2.

### Example 8

Preparation and evaluation were performed according to the method of Example 7, except that in step (1), the X-type molecular sieve was placed in a water vapor atmosphere of a mixture of water vapor and nitrogen at a water vapor partial pressure of 0.9 bar and treated at a temperature of 500°C for 2 hours. A modified X-type molecular sieve sample h was obtained; thereby an adsorbent H was prepared.

The surface area of the modified X-type molecular sieve sample h measured by nitrogen physical adsorption was 10.4m²/g, the micropore volume was 0.307 g/mL, the total pore volume was 0.343 g/mL, and the sum of the mesopore and macropore volumes accounted for 10.5% of the total pore volume.

The adsorption selectivity, PX half-peak width and toluene adsorption capacity of the adsorbent H are shown in Table 2.

### Example 9-1

Preparation and evaluation were performed according to the method of Example 7, except that in step (2), an alkaline solution having a NaOH concentration of 1.6 mol/L, a SiO₂ concentration of 12.0 g/L, and a dodecyltrimethylammonium chloride concentration of 0.01 mol/L was used to treat the first product obtained in step (1). A modified X-type molecular sieve sample i-1 was obtained; thereby adsorbent I-1 was prepared.

The surface area of the modified X-type molecular sieve sample i-1 measured by nitrogen physical adsorption was 12.6 m²/g, the micropore volume was 0.327 g/mL, the total pore volume was 0.365 g/mL, and the sum of the mesopore and macropore volumes accounted for 10.4% of the total pore volume.

The adsorption selectivity, PX half-peak width and toluene adsorption capacity of adsorbent I-1 are shown in Table 2.

### Example 9-2

The preparation and evaluation were performed according to the method of Example 7, except that in step (2), an alkaline solution having a NaOH concentration of 1.6 mol/L, a SiO₂ concentration of 12.0 g/L, and a dodecyltrimethylammonium chloride concentration of 0.03 mol/L was used to treat the first product obtained in step (1). A modified X-type molecular sieve sample i-2 was obtained; thereby adsorbent I-2 was obtained.

The surface area of the modified X-type molecular sieve sample i-2 measured by nitrogen physical adsorption was 13.2 m²/g, the micropore volume was 0.322 g/mL, the total pore volume was 0.375 g/mL, and the sum of the mesopore and macropore volumes accounted for 14.1% of the total pore volume.

The adsorption selectivity, PX half-peak width and toluene adsorption capacity of the adsorbent I-2 are shown in Table 2.

### Comparative Example 9-3

The preparation and evaluation were performed according to the method of Example 7, except that in step (2), an alkaline solution having a NaOH concentration of 1.6 mol/L, a SiO₂ concentration of 12.0 g/L, and a dodecyltrimethylammonium chloride concentration of 0 mol/L was used to treat the first product obtained in step (1). The modified X-type molecular sieve sample db3 was obtained; thereby adsorbent DB3 was obtained.

The surface area of the modified X-type molecular sieve sample db3 measured by nitrogen physical adsorption was 5.8 m²/g, the micropore volume was 0.325g/mL, the total pore volume was 0.344g/mL, and the sum of the mesopore and macropore volumes accounted for 5.5 % of the total pore volume.

The adsorption selectivity, PX half-peak width and toluene adsorption capacity of the adsorbent DB3 are shown in Table 2.

### Example 10

Preparation and evaluation were performed according to the method of Example 7, except that in step (2), after being dried at 100°C for 5 hours, and then calcined at a constant temperature of 150°C for 1.0 hours, 260°C for 1.0 hours, 380°C for 1.0 hours, 450°C for 1.0 hours, and 550°C for 4.0 hours, a modified X-type molecular sieve sample j was obtained; thereby adsorbent J was obtained.

The surface area of the modified X-type molecular sieve sample j measured by nitrogen physical adsorption was 16.8m²/g, the micropore volume was 0.325 g/mL, the total pore volume was 0.377 g/mL, and the sum of the mesopore and macropore volumes accounted for 13.9% of the total pore volume.

The adsorption selectivity, PX half-peak width and toluene adsorption capacity of the adsorbent J are shown in Table 2.

### Example 11

Preparation and evaluation were performed according to the method of Example 7, except that in step (2), 390 L of an alkaline solution having a NaOH concentration of 1.2 mol/L, a SiO₂ concentration of 8.0 g/L, and a hexadecyltrimethylammonium chloride concentration of 0.003 mol/L was used to treat the first product obtained in step (1). Modified X-type molecular sieve sample k was obtained; thereby adsorbent K was obtained.

The surface area of the modified X-type molecular sieve sample k measured by nitrogen physical adsorption was 11.2m²/g, the micropore volume was 0.320 g/mL, the total pore volume was 0.354 g/mL, and the sum of the mesopore and macropore volumes accounted for 9.6% of the total pore volume.

The adsorption selectivity, PX half-peak width and toluene adsorption capacity of the adsorbent K are shown in Table 2.

### Example 12

The preparation and evaluation were performed according to the method of Example 7, except that in step (2), the modified X-type molecular sieve sample 1 was obtained after calcination at a constant temperature of 200°C for 1.5 hours, 300°C for 1.5 hours, and 550°C for 3.0 hours. Adsorbent L was obtained.

The surface area of the modified X-type molecular sieve sample 1 measured by nitrogen physical adsorption was 15.6 m²/g, the micropore volume was 0.307 g/mL, the total pore volume was 0.360 g/mL, and the sum of the mesopore and macropore volumes accounted for 14.7% of the total pore volume.

The adsorption selectivity, PX half-peak width and toluene adsorption capacity of adsorbent L are shown in Table 2.

### Comparative Example 4

Preparation and evaluation were performed according to the method of Example 7, except that the first product obtained in step (1) was not subjected to the treatment of step (2), a modified X-type molecular sieve comparative sample db4 was obtained. Thereby adsorbent DB4 was obtained.

The surface area of the modified X-type molecular sieve comparative sample db4 measured by nitrogen physical adsorption was 25.4m²/g, the micropore volume was 0.256 g/mL, the total pore volume was 0.306 g/mL, and the sum of the mesopore and macropore volumes accounted for 16.3% of the total pore volume.

The adsorption selectivity, PX half-peak width and toluene adsorption capacity of adsorbent DB4 are shown in Table 2.

### Comparative Example 5

Preparation and evaluation were performed according to the method of Example 7, except that the X-type molecular sieve (having a SiO₂/Al₂O₃ molar ratio of 2.43, a particle size of 1.0 µm, a micropore volume of 0.335 g/mL, a total pore volume of 0.351 g/mL, and a surface area of 5.5 m²/g, the sum of pore volume of mesopores and macropores accounting for 4.3% of the total pore volume) was not subjected to the treatment of step (1) but directly processed in step (2) to obtain the modified X-type molecular sieve comparative sample db5. Thereby adsorbent DB5 was obtained.

The surface area of the modified X-type molecular sieve comparative sample db5 measured by nitrogen physical adsorption was 8.6m²/g, the micropore volume was 0.328 g/mL, the total pore volume was 0.347 g/mL, and the sum of the mesopore and macropore volumes accounted for 5.5% of the total pore volume.

The adsorption selectivity, PX half-peak width and toluene adsorption capacity of adsorbent DB5 are shown in Table 2.

### Comparative Example 6

Preparation and evaluation were performed according to the method of Example 7, except that X type molecular sieve (having a SiO₂/Al₂O₃ molar ratio of 2.43, a particle size of 1.0 µm, a micropore volume of 0.335 g/mL, a total pore volume of 0.351 g/mL, and a surface area of 5.5 m²/g, the sum of mesopore and macropore volumes accounting for 4.6% of the total pore volume) was not subjected to the treatment of steps (1) and (2). The adsorption selectivity, PX half-peak width and toluene adsorption capacity of the obtained adsorbent DB6 are shown in Table 2.

**Table 2**

| Example | Adsorbent sample | Adsorption selectivity | | | W_{1/2}, mL | Adsorption capacity, mg/g |
|---|---|---|---|---|---|---|
| | | β_{P/E} | β_{P/M} | β_{P/O} | | |
| Example 7 | G | 1.73 | 4.41 | 3.92 | 12.62 | 184 |
| Example 8 | H | 1.71 | 4.49 | 4.15 | 11.17 | 177 |
| Example 9-1 | I-1 | 1.72 | 4.67 | 4.23 | 11.60 | 185 |
| Example 9-2 | I-2 | 1.72 | 4.59 | 4.19 | 12.02 | 183 |
| Comparative Example 9-3 | DB3 | 1.71 | 4.21 | 3.43 | 14.45 | 184 |
| Example 10 | J | 1.73 | 4.52 | 4.17 | 12.69 | 184 |
| Example 11 | K | 1.73 | 4.48 | 4.08 | 12.10 | 182 |
| Example 12 | L | 1.71 | 4.23 | 3.84 | 12.99 | 178 |
| Comparative Example 4 | DB4 | 1.40 | 2.82 | 1.99 | 17.05 | 137 |
| Comparative Example 5 | DB5 | 1.70 | 4.10 | 3.23 | 14.79 | 186 |
| Comparative Example 6 | DB6 | 1.71 | 4.19 | 3.42 | 14.62 | 190 |

In Table 2, W_{1/2} is a direct reflection of the mass transfer performance. The smaller W_{1/2} is, the better the mass transfer performance is. Therefore, the mass transfer performance of Examples 7 to 12 is significantly better than that of Comparative Examples 4 to 6. Example 12 uses three-stage calcination, which makes the rate of temperature increase too fast compared to the four-stage or five-stage calcination of Examples 7 to 11, so that the local high temperature formed by the combustion of the organic amine formed by the organic ammonium salt will destroy the X-type molecular sieve, resulting in reduced mass transfer performance and adsorption selectivity.

The adsorption selectivity in Table 2 is affected by the combined effects of mass transfer performance and micropore adsorption capacity. Comparative Example 4 uses only water vapor treatment, the X-type molecular sieve is damaged, the crystallinity is reduced, and a large number of amorphous species are formed. These amorphous species will not only block the mesopores, but also cause the micropore adsorption capacity to be significantly reduced. Therefore, the prepared adsorbent has the worst adsorption selectivity. Comparative Example 5 uses only alkaline solution to treat the X-type molecular sieve without water vapor treatment, so that the proportion of the sum of the mesopore and macropore volumes to the total pore volume increases less than that of the unmodified X-type molecular sieve, but the calcination process causes the X-type molecular sieve to be slightly damaged, resulting in a decrease in the adsorption capacity and mass transfer performance of the prepared adsorbent, and the adsorption selectivity also decreases accordingly. Comparative Example 6 is an unmodified X-type molecular sieve; due to the small number of mesopores and macropore structures thereof, the mass transfer performance of the prepared adsorbent is low, and the adsorption selectivity is also low. By comparing Examples 7, 9-1, 9-2 with Comparative Example 9-3, it can be seen that when using an alkaline solution to treat the X-type molecular sieve, the presence of a relatively low content of an organic ammonium salt in the alkaline solution is necessary and important. The organic ammonium salt can increase the ratio of mesopores and macropores volumes, thereby significantly improving the mass transfer performance of the modified X-type molecular sieve. However, an excessively high content of the organic ammonium salt in the alkaline solution has a negative impact on the mass transfer performance and adsorption capacity of the modified X-type molecular sieve.

### Example 13

The adsorbent G was used to separate p-xylene in a small simulated moving bed with continuous countercurrent flow.

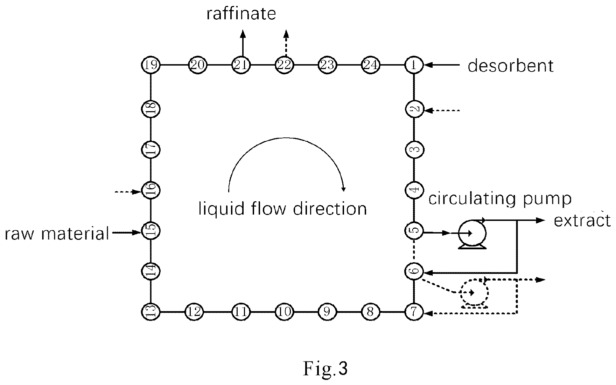

The small simulated moving bed device included 24 adsorption columns connected in series, each column was 195 mm long, the inner diameter of the column was 30 mm, and the total loading amount of the adsorbent was 3300 mL. The 24 columns connected in series were connected at both ends by a circulating pump to form a closed loop, as shown in Figure 3. The 24 adsorption columns were divided into four sections by the four streams of inlet and outlet materials: adsorption raw material, desorbent, extract, and raffinate; that is, the 7 adsorption columns between the adsorption raw material (column 15) and the raffinate (column 21) were the adsorption zone, the 9 adsorption columns between the extract (column 6) and the adsorption raw material (column 14) were the purification zone, the 5 adsorption columns between the desorbent (column 1) and the extract (column 5) were the desorption zone, and the 3 adsorption columns between the raffinate (column 22) and the desorbent (column 24) were the buffer zone. The temperature of the entire adsorption system was controlled to 177°C and the pressure was 0.8MPa.

During the operation, the desorbent p-diethylbenzene and the raw material were continuously injected into the simulated moving bed at a flow rate of 1420 mL/h and 2010 mL/h respectively, and the extract and the raffinate were drawn out of the device at a flow rate of 895 mL/h and 2535 mL/h respectively. The composition of the raw material was: 9.3% by mass of ethylbenzene, 18.5% by mass of p-xylene, 45.5% by mass of m-xylene, 17.4% by mass of o-xylene, and 9.4% by mass of non-aromatic components.

The flow rate of circulation pump was set to 3720 mL/h, and every 50 seconds, the four streams of material moved one adsorption column simultaneously in the same direction as the liquid flow (moving from the solid line to the dotted line in Figure 3, and so on). Under stable operating conditions, the purity of p-xylene obtained by adsorbent G was 99.83% by mass, and the yield was 98.33% by mass.

### Example 14

Adsorbent H was loaded in the small simulated moving bed device, and an adsorptive separation experiment of p-xylene was carried out according to the method of Example 13. The purity of p-xylene obtained under stable operating state was 99.82% by mass, and the yield was 98.15% by mass.

### Comparative Example 7

Adsorbent DB4 was loaded in the small simulated moving bed device, and an adsorptive separation experiment of p-xylene was carried out according to the method of Example 13. The purity of p-xylene obtained under stable operating state was 82.26% by mass, and the yield was 80.65% by mass.

### Comparative Example 8

Adsorbent DB5 was loaded in the small simulated moving bed device, and an adsorptive separation experiment of p-xylene was carried out according to the method of Example 13. The purity of p-xylene obtained under stable operating state was 99.50% by mass, and the yield was 90.33% by mass.

### Comparative Example 9

Adsorbent DB6 was loaded in the small simulated moving bed device, and an adsorptive separation experiment of p-xylene was carried out according to the method of Example 13. The purity of p-xylene obtained under stable operating state was 99.56% by mass, and the yield was 93.62% by mass.

It can be seen from the above Example 14 and Comparative Examples 7, 8 and 9 that the use of the adsorbent according to the present invention in the small-scale simulated moving bed device can achieve significantly higher p-xylene purity and significantly higher p-xylene yield than the use of the comparative adsorbent.

## Claims

1. A modified X-type molecular sieve, **characterized in that**, as measured by nitrogen physical adsorption method, its surface area is 9.5-19 m²/g, preferably 10-18 m²/g, and the sum of the mesopore and macropore volumes accounts for 6-18%, preferably 8%-15% of the total pore volume; wherein the mesopores are pores with a pore diameter of 10nm to 50nm, and the macropores are pores with a pore diameter of greater than 50nm and less than 80nm.

2. The modified X-type molecular sieve according to claim 1, **characterized in that**, in the modified X-type molecular sieve, the SiO₂/Al₂O₃ molar ratio is 2.21-2.55, preferably 2.25-2.4.

3. The modified X-type molecular sieve according to claim 1 or 2, **characterized in that** the micropore volume of the modified X-type molecular sieve is 0.290-0.340 g/ml, preferably 0.300-0.330 g/ml, and the total pore volume is 0.340-0.390 g/ml, preferably 0.350-0.380 g/ml; preferably, in the modified X-type molecular sieve, the amorphous species content is 2-12% by mass, more preferably 3.0-8.0% by mass.

4. A preparation method of a modified X-type molecular sieve, in particular the modified X-type molecular sieve according to any one of claims 1 to 3, **characterized in that** it comprises the following steps:
(1) placing the X-type molecular sieve in a water vapor atmosphere to conduct hydrothermal treatment, preferably at a temperature of 250-550 °C for at least 0.5 hours, and then washing and drying it to obtain a first product;
(2) placing the first product obtained in step (1) in an aqueous solution containing NaOH, SiO₂ and an organic ammonium salt, preferably at a temperature of 60-120 °C for at least 0.5 hours, washing and then drying and calcining it to obtain the modified X-type molecular sieve.

5. The preparation method according to claim 4, **characterized in that** the water vapor atmosphere in step (1) comprises water vapor and an inert gas or air, preferably the inert gas is selected from nitrogen, carbon dioxide and argon, preferably, the water vapor partial pressure in the water vapor atmosphere is 0.6-1.0 bar; preferably, the treatment time in the water vapor atmosphere is at least 1 hour, more preferably at least 2 hours.

6. The preparation method according to any one of claims 4-5, **characterized in that**, in the aqueous solution containing NaOH, SiO₂ and organic ammonium salt in step (2), the NaOH concentration is 1.0-2.0 mol/L, preferably 1.2-1.8 mol/L; the SiO₂ concentration is 5.0-20.0 g/L, preferably 6.0-18.0 g/L; and the organic ammonium salt concentration is 0.0005-0.05 mol/L, preferably 0.001-0.04 mol/L.

7. The preparation method according to any one of claims 4-6, **characterized in that** in step (2), the ratio of the aqueous solution containing NaOH, SiO₂ and an organic ammonium salt to the first product is 2-5:1, preferably 3-4:1, wherein the aqueous solution containing NaOH, SiO₂ and an organic ammonium salt is measured in milliliter, and the first product is measured in gram.

8. The preparation method according to any one of claims 4-7, **characterized in that** the total carbon number of the organic ammonium salt is 9-25, preferably 11-20, and preferably is selected from one or more of dodecyltrimethylammonium chloride, tetradecyltrimethylammonium chloride, hexadecyltrimethylammonium chloride, and octadecyltrimethylammonium chloride.

9. The preparation method according to any one of claims 4-8, **characterized in that** in step (2), the calcination is a n-stage calcination, wherein n≥4, preferably the constant temperature time of each stage is ≥1.0 hour, and preferably the maximum temperature of each stage does not exceed 550°C.

10. The preparation method according to any one of claims 4 to 9, **characterized in that** the X-type molecular sieve used in step (1) has a SiO₂/Al₂O₃ molar ratio of 2.0-2.5, and a particle size of 0.6-2.5 µm; preferably, the X-type molecular sieve used has a SiO₂/Al₂O₃ molar ratio of 2.25-2.40, and a particle size of 0.8-1.5 µm.

11. The preparation method according to any one of claims 4 to 10, **characterized in that** in step (2), the first product is treated in the aqueous solution containing NaOH, SiO₂ and an organic ammonium salt for at least 1 hour, preferably at least 1.5 hours, and treatment temperature is 60-120 °C, preferably 70-100 °C.

12. An adsorptive separation method for aromatic hydrocarbon isomers, **characterized in that** the modified X-type molecular sieve according to one of claims 1-3 is used as an adsorbent, and preferably the aromatic hydrocarbon isomers are selected from ethylbenzene, p-xylene, o-xylene and m-xylene.

13. An adsorbent for aromatic hydrocarbon isomers, in particular an adsorbent for p-xylene, **characterized in that** it comprises the modified X-type molecular sieve according to any one of claims 1 to 3 or prepared by the method according to any one of claims 4 to 9, preferably the adsorbent comprises 88-95% by mass of the modified X-type molecular sieve.

14. The adsorbent for aromatic hydrocarbon isomers according to claim 13, **characterized in that** it also contains 4-11% by mass of transformed crystal X-type molecular sieve and 1-3% by mass of matrix.

15. The adsorbent for aromatic hydrocarbon isomers according to any one of claims 13 to 14, **characterized in that** the adsorption capacity measured by toluene dynamic adsorption method is 175-186 mg/g, preferably 177-184 mg/g.

16. A preparation method of the adsorbent for aromatic hydrocarbon isomers according to any one of claims 13 to 14, **characterized in that** it comprises the following steps:
(1) placing the X-type molecular sieve in a water vapor atmosphere to conduct hydrothermal treatment, then washing and drying it to obtain a first product;
(2) treating the first product obtained in step (1) in an aqueous solution containing NaOH, SiO₂ and an organic ammonium salt, then washing, drying and calcining it to obtain the modified X-type molecular sieve;
(3) mixing and forming the modified X-type molecular sieve obtained in step (2) with a binder, then calcining it to obtain a formed body;
(4) treating the formed body obtained in step (3) with an alkaline solution to in-situ crystallize the binder therein, and then drying it;
(5) subjecting the product obtained in step (4) to barium ion exchange and optionally potassium ion exchange, then drying and activating it to obtain the adsorbent for aromatic hydrocarbon isomers.

17. The preparation method according to claim 16, **characterized in that** it comprises the following steps:
(1) placing the X-type molecular sieve in a water vapor atmosphere to conduct hydrothermal treatment at a temperature of 250-550 °C for at least 0.5 hours, and then washing and drying it to obtain a first product;
(2) placing the first product obtained in step (1) in an aqueous solution containing NaOH, SiO₂ and an organic ammonium salt to conduct treatment at a temperature of 60-120 °C for at least 0.5 hours, then washing, drying and calcining it to obtain the modified X-type molecular sieve;
(3) forming the modified X-type molecular sieve obtained in step (2) with a binder, drying and calcining it to obtain a formed body, wherein the shape of the formed body is preferably a small ball;
(4) treating the formed body obtained in step (3) with a mixed solution of sodium hydroxide and potassium hydroxide to in-situ crystallize the binder therein, and then drying it;
(5) subjecting the dried formed body obtained in step (4) to cation exchange with a solution of soluble barium salt or a solution of soluble barium salt and soluble potassium salt, then drying and activating it.

18. The preparation method according to any one of claims 16-17, **characterized in that**, in step (3), the mass ratio of the modified X-type molecular sieve to the binder is 88-95:5-12; preferably, the amount of water added is 6-22% by mass of the total amount of solid powder; preferably, the drying temperature is 80-150°C; preferably, the drying time is 0.5-20 hours; preferably, the calcination temperature is 500-700°C; preferably, the calcination time is 0.5-6.0 hours.

19. The preparation method according to any one of claims 16 to 18, **characterized in that** in step (4), the hydroxide ion concentration in the mixed solution is 0.1-2.0 mol/L, and the K/(Na+K) molar ratio is 0.1-0.6; preferably, the temperature of in-situ crystallization treatment is 80-100°C; preferably, the time of in-situ crystallization treatment is 1.5-6.0 hours.

20. The preparation method according to any one of claims 16-18, **characterized in that** in step (5), the soluble barium salt is selected from barium nitrate and barium chloride, and the soluble potassium salt is selected from potassium nitrate and potassium chloride, preferably the drying temperature is 60-130°C, preferably 70-120°C, and the drying time is 1.0-24.0 hours, preferably 3-15 hours; preferably the activating is carried out in a nitrogen atmosphere at 50-120°C, preferably 60-100°C, for 5-30 hours.

21. A adsorptive separation method for aromatic hydrocarbon isomers, **characterized in that** it uses the adsorbent according to any one of claims 13 to 15, preferably the aromatic hydrocarbon isomers are selected from ethylbenzene, p-xylene, o-xylene and m-xylene.
